# DEMANDE DE BREVET EUROPEEN

(11) **EP 1 269 989 A1**
(43) Date de publication de la demande: **02.01.2003**
(21) Numéro de dépôt: 02291598.7
(22) Date de dépôt: 26.06.2002
(51) Int. Cl.: A61K 7/48

(54) **Composition cosmétique ou dermatologique comprenant des dérivés d'acylaminoamides**

(30) Priorité: 26.06.2001 FR 0108434
(71) Demandeur: L'OREAL, 75008 Paris (FR)
(72) Inventeur: Breton, Lionel, 78000 Versailles (FR); Mahe, Yann, 91390 Morsang sur Orge (FR)
(74) Mandataire: Catherine, Alain

(57) **Abrégé**

Composition cosmétique ou dermatologique caractérisée en ce qu'elle comprend une association entre un composé inhibiteur de l'élastase de la famille des N-Acylaminoamides et au moins un au moins un composé anti-inflammatoire.

## Description

La présente invention se rapporte au domaine des compositions cosmétiques ou dermatologiques. Elle est relative à de nouvelles compositions cosmétiques ou dermatologiques comprenant une association entre un composé inhibiteur de l'élastase de la famille des N-Acylaminoamides et au moins un composé anti-inflammatoire. Cette composition est préférentiellement destinée à améliorer les signes cutanés du vieillissement et/ou du photovieillissement en ralentissant les altérations du tissu conjonctif et en améliorant l'état fonctionnel de la peau, et notamment les signes associés ou résultant d'un processus inflammatoire, en particulier d'un processus micro-inflammatoire chronique.

La peau humaine est constituée de deux compartiments à savoir un compartiment superficiel, l'épiderme, et un compartiment profond, le derme. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également traversé par des vaisseaux sanguins et des fibres nerveuses.

On sait que lors d'un stress cutané superficiel, qui peut notamment être d'origine chimique, physique ou bactérienne, les kératinocytes des couches superficielles de l'épiderme libèrent des médiateurs biologiques qui possèdent la capacité d'attirer certaines cellules infiltrantes de la peau, elles-mêmes responsables de l'entretien d'une irritation locale transitoire.

Parmi les médiateurs biologiques pouvant être produits par les kératinocytes ainsi stressés, on citera les chimiokines qui sont des cytokines chimioattractives responsables du recrutement de leucocytes sur les sites inflammatoires, dont l'interleukine 8 (IL-8) qui est plus particulièrement responsable du recrutement des neutrophiles.

Ces cellules infiltrant les zones irritées ou agressées libèrent alors des enzymes parmi lesquelles on peut citer l'élastase leucocytaire.
Sous l'action de cette enzyme notamment, les fibres élastiques de soutien extracellulaire du tissu conjonctif peuvent être dégradées, et entraîner ainsi une diminution de l'élasticité de la peau.

Il est même par ailleurs connu qu'en synergie avec la cathepsine G, l'élastase leucocytaire peut dissocier l'intégrité de l'épiderme en élargissant les espaces intercellulaires interkératinocytaires.

Ainsi, à long terme, la somme des micro-stress cutanés superficiels, par exemple générés par une exposition prolongée aux UV ou par des agents irritants, peut entraîner une perte plus ou moins accélérée de l'élasticité naturelle de la peau. Le réseau formé par les fibres élastiques du tissu conjonctif sous-jacent et des espaces extracellulaires peut alors progressivement être destructuré. Il s'en suit un vieillissement accéléré de la peau (peau ridée et/ou moins souple) via l'altération du réseau élastique dermique, ainsi qu'une accentuation des rides (rides plus profondes).

Lors d'un stress cutané (chimique, physique, bactérien ou neurogène) les kératinocytes libèrent des médiateurs biologiques (appelés facteurs chimioattractants) qui possèdent la capacité d'attirer certaines cellules inflammatoires du compartiment sanguin vers le tissu cutané . Ces cellules sont responsables de la genèse puis de l'entretien d'une irritation locale.

Parmi les facteurs chimio-attractants pouvant être produits par les kératinocytes stressés, l'interleukine 8 (IL-8) est plus spécifiquement responsables du recrutement des polynucléaires neutrophiles. Ces cellules infiltrant les zones irritées ou agressées libèrent alors des enzymes parmi lesquelles l' élastase leucocytaire .

Sous l'action de cette enzyme, les fibres élastiques de soutien extracellulaire du tissu conjonctif sont dégradées. En synergie avec la cathepsine G, l'élastase leucocytaire peut également dissocier l'intégrité de l'épiderme en élargissant les espaces intercellulaires inter-kératinocytaires (Ludolph-Hauser et al *Exp. Dermatol.* 1999 8(1) 46-52). L'élastase leucocytaire a récemment été incriminée dans l'entretien des escarres et la survenue des ulcères veineux des jambes, via son activité de dégradation de la fibronectine (Herrick S et al *Lab.lnvest.* 1997(3) 281-288. La somme des micro-stress localisés de dégradation (suite par exemple à une exposition prolongée au soleil) peut aboutir au long terme à une perte accélérée de l'élasticité naturelle de la peau. Le réseau des fibres élastiques du tissu conjonctif sous jacent et des espaces extracellulaires est alors progressivement déstructuré. Cette dégradation accélérée peut se cumuler avec le processus de vieillissement normal de la peau qui se caractérise par une plus grande sensibilité des fibres élastiques à l'action de l'élastase (Stadler R & Orfanos CE *Arch. Dermatol.* Res. 1978 262 (1) 97-111.

Il est connu dans l'état de la technique d'apporter, dans le tissu cutané, des molécules capables de ralentir l'activité de dégradation des fibres élastiques des espaces intercellulaires.

La solution technique selon l'invention consiste à apporter , en plus de l'élément régulateur en aval de l'activité élastasique (i.e le dérivé N-Acylaminoamide inhibiteur de l'activité enzymatique de l' élastase leucocytaire, l' acide {2-[acétyl-(3-trifluorométhyl-phényl)-amino]-3-méthyl-butyrylamino} acétique) un ou plusieurs actifs capables de réguler aussi les événement inflammatoires précoces plus en amont. Selon un mode de réalisation, le produit à associer dans la formule empêche la cascade de production des cytokines pro-inflammatoires comme l'IL-8, le TNF ou l'IL-1.

En conséquence, l'invention a pour objet une composition cosmétique ou dermatologique caractérisée en ce qu'elle comprend une association entre un composé inhibiteur de l'élastase de la famille des N-Acylaminoamides et au moins un composé anti-inflammatoire.

Un autre objet de l'invention consiste en un procédé de traitement cosmétique de la peau du corps ou du visage, y compris le cuir chevelu, dans lequel on applique sur la peau une composition cosmétique telle que définie ci-dessus.

Il a en effet été constaté que les composés N-acylaminoamides, et notamment les composés de formule (I) présentaient une activité inhibitrice de l'activité des élastases, et qu'ils pouvaient donc être employés pour limiter et/ou combattre la dégradation des fibres élastiques.
Il s'en suit qu'ils peuvent être employés dans ou pour la préparation d'une composition, les composés ou la composition étant destinés à traiter, de manière préventive et/ou curative, les signes cutanés du vieillissement.

L'association nouvelle des composés N-Acylaminoamides avec au moins un composé anti-inflammatoire permet de renforcer significativement l'effet antivieillissement du tissu matriciel, par l'apport d'un effet anti-inflammatoire.

La composition obtenue est destinée à traiter les désordres du vieillissement et/ou à être plus spécifiquement destinée à traiter tous les désordres cutanés associés à une inflammation, en particulier à un processus micro-inflammatoire chronique.

Préférentiellement, cette nouvelle association est utilisée dans des préparations cosmétiques de soin et/ou d'hygiène des zones exposées au soleil (scalp, corps, visage, lèvres), dans les préparations cosmétiques de soin et/ou d'hygiène des zones ulcérées, dans les dentifrices ou lotions de rinçage buccales et, de façon générale, dans toutes les préparations cosmétiques dites " anti-vieillissement cutané " qui ont pour objectif de ralentir la destructuration chronobiologique des tissus de soutien et de l'architecture des éléments matriciels cutanés. Plus particulièrement, cette association sera réservée aux peaux comédogènes et ou acnéiques.
Cette nouvelle association est aussi utilisée dans des préparations cosmétiques de soin des zones exposées au soleil (scalp, corps, visage, lèvres), dans les préparation cosmétiques de soin des zones ulcérées, dans les dentifrices ou lotions de rinçage buccales et, de façon générale, dans toutes les préparations cosmétiques dites "anti-vieillissement cutané " qui ont pour objectif de ralentir la destructuration chronobiologique des tissus de soutien et de l'architecture des éléments matriciels cutanés .

Sans vouloir être lié par une quelconque théorie, le demandeur considère que le fait d'apporter, au niveau des kératinocytes des couches superficielles de la peau, des composés susceptibles de ralentir l'activité de dégradation des fibres élastiques des espaces intercellulaires, peut permettre de diminuer ce phénomène de vieillissement accéléré de la peau, dû à des stress cutanés superficiels et que l'association de ces composés avec un composé anti-inflammatoire renforce considérablement leurs effets.

### Composés N-acylamino-amides préférés préférentiellement

Les composés susceptibles d'être employés préférentiellement dans la présente invention répondent donc à la formule (I) suivante : 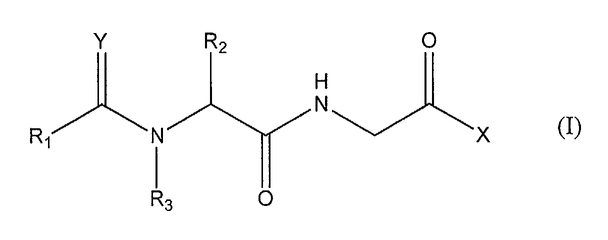 dans laquelle :
- le radical Y représente O ou S,
- le radical R1 représente :
   - (i) un atome d'hydrogène;
   - (ii) un radical hydrocarboné, linéaire, ramifié ou cyclique, saturé ou insaturé, ayant 1 à 18 atomes de carbone,
      éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR; -P(O)-(OR)₂; -SO₂-OR;
      avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
      lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
   - (iii) un radical choisi parmi les radicaux -OR; -NH₂; -NHR; -NRR'; -NH-COR; -COOR; -COR ;
      avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
      lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R2 représente un radical hydrocarboné, linéaire, ramifié ou cyclique, saturé ou insaturé, ayant 1 à 18 atomes de carbone,
   éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR;
   avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
   lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R3 représente un radical choisi parmi ceux de formule (II) ou (III):

   (II) -A-C₆H_{(5-y)}-B_{y}

   (III) -C₆H_{(5-y')}-B_{y'}

   dans lesquelles :
   - y est un entier compris entre 0 et 5 inclus, et y' est un entier compris entre 1 et 5 inclus;
   - A est un radical divalent hydrocarboné, linéaire ou ramifié, saturé ou insaturé, ayant 1 à 18 atomes de carbone,
      éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
      lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
   - B est un radical hydrocarboné, linéaire ou ramifié, saturé ou insaturé, ayant 1 à 18 atomes de carbone,
      éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
      lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical X représente un radical choisi parmi -OH, -OR₄, -NH₂, -NHR₄, -NR₄R₅, -SR₄, -COOR₄; -COR₄;
   avec R₄ et R₅ représentant, indépendamment l'un de l'autre, un radical hydrocarboné, linéaire, cyclique ou ramifié, saturé ou insaturé, ayant 1 à 6 atomes de carbone, éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné; lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
   lesdits radicaux R₄ et R₅ pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné.

Sont également compris dans cette définition, les sels d'acide minéral ou organique desdits composés, ainsi que leurs isomères optiques, sous forme isolée ou en mélange racémique.

Par radical hydrocarboné, linéaire, cyclique ou ramifié, on entend notamment les radicaux de type alkyle, aryle, aralkyle, alkylaryle, alcényle, alcynyle.

Le groupement C₆H₅ présent dans le radical R3 doit être compris comme un groupement cyclique aromatique.

De préférence, le radical Y représente l'oxygène.

De préférence, le radical R1 représente l'hydrogène ou un radical hydrocarboné, linéaire ou ramifié, saturé ou insaturé, ayant 1 à 12, et notamment 1, 2, 3, 4, 5 ou 6, atomes de carbone, éventuellement substitué. Notamment, les substituants peuvent être choisis parmi -OH, -OR et/ou -P(O)-(OR)₂ avec R représentant un radical hydrocarboné, linéaire, ramifié ou cyclique, saturé ou insaturé, ayant 1 à 6 atomes de carbone, éventuellement halogéné, voire perhalogéné.

Préférentiellement, le radical R1 représente un radical méthyle, éthyle, propyle ou isopropyle, éventuellement substitué par un groupement -OH ou -P(O)-(OR)₂ avec R représentant méthyle, éthyle, propyle ou isopropyle.

De préférence, le radical R2 représente un radical hydrocarboné, linéaire, ramifié ou cyclique, saturé ou insaturé, ayant 1 à 12, notamment 1, 2, 3, 4, 5 ou 6, atomes de carbone, éventuellement substitué.
Notamment, les substituants peuvent être choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné.

Préférentiellement, le radical R2 représente un radical méthyle, éthyle, propyle, isopropyle, n-butyle, ter-butyle ou isobutyle.

De préférence, le radical R3 représente un radical de formule -C₆H_{(5-y')}-B_{y'} pour lequel y' = 1, 2 ou 3; ou un radical de formule -A-C₆H_{(5-y)}-B_{y} pour lequel y = 0, 1 ou 2.

De préférence, A est un radical divalent hydrocarboné, linéaire ou ramifié, saturé ou insaturé, ayant 1 à 12 atomes de carbone, éventuellement substitué .

Les substituants de A sont de préférence choisis parmi -Hal (halogène, voire perhalogène); -CN; -COOR; -NO₂; -SO₂-OR; avec R représentant un radical hydrocarboné, linéaire, ramifié ou cyclique, saturé ou insaturé, ayant 1 à 6 atomes de carbone, éventuellement halogéné, voire perhalogéné.

De préférence, B est un radical hydrocarboné, linéaire ou ramifié, saturé ou insaturé, ayant 1 à 12 atomes de carbone, éventuellement substitués.

Les substituants de B sont de préférence choisis parmi -Hal (halogène, voire perhalogène); -CN; -COOR; -NO₂; -SO₂-OR; avec R représentant un radical hydrocarboné, linéaire, ramifié ou cyclique, saturé ou insaturé, ayant 1 à 6 atomes de carbone, éventuellement halogéné, voire perhalogéné.

Préférentiellement, le radical R3 représente un groupement choisi parmi l'une des formules suivantes : 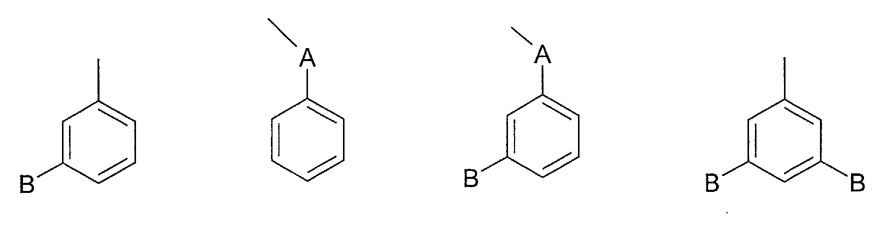 dans lesquelles A et B ont les significations ci-dessus.

Notamment, le radical divalent A peut être un méthylène, un éthylène, un propylène.

Le radical B est de préférence un radical méthyle, éthyle, propyle ou isopropyle, substitué par un ou plusieurs halogènes, notamment chlore, brome, iode ou fluor, et préférentiellement totalement halogéné (perhalogéné), tel que perfluoré. On peut en particulier citer le radical perfluorométhyle (-CF3) comme tout particulièrement préféré.

De préférence, le radical X représente un radical choisi parmi -OH ou -OR₄ avec R₄ représentant un radical hydrocarboné, linéaire, cyclique ou ramifié, saturé ou insaturé, ayant 1 à 6 atomes de carbone, éventuellement substitué.

Les substituants peuvent être choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné.

Préférentiellement, le radical X représente un radical choisi parmi -OH, -OCH₃, -OC₂H₅, -O-C₃H₇ ou -OC₄H₉.

Parmi les composés particulièrement préférés, on peut citer :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle,
- le (2-{benzyl-[(diethoxy-phosphoryl)-acétyl]-amino}-3-méthyl-butyrylamino) acétate d'éthyle.

Les composés selon l'invention peuvent être aisément préparés par l'homme du métier sur base de ses connaissances générales. On peut notamment faire réagir ensemble un acide carboxylique, un aldéhyde, un composé aminé et un isonitrile, selon la réaction de Ugi.

Bien entendu, lors de la synthèse des composés selon l'invention, et en fonction de la nature des différents radicaux présents sur les composés de départ, l'homme du métier pourra veiller à protéger certains substituants pour que ceux-ci n'interviennent pas dans la suite des réactions.

La quantité de composé à utiliser dans les compositions selon l'invention peut être aisément déterminée par l'homme du métier, en fonction de la nature du composé utilisé, de la personne à traiter et/ou de l'effet recherché. D'une manière générale, cette quantité peut être comprise entre 0,00001 et 20% en poids par rapport au poids total de la composition, préférentiellement entre 0,001 et 10% en poids.

### Composés anti-inflammatoires préférés

Par " composé " ou "agent" anti-inflammatoire selon l'invention, on entend notamment un composé antagoniste des cytokines inflammatoires. Un antagoniste des cytokines inflammatoires est un composé susceptible d'inhiber la synthèse et/ou la libération d'une ou plusieurs cytokines inflammatoires. Entrent également dans la définition d'un antagoniste des cytokines inflammatoires, les composés qui inhibent ou bloquent la fixation de ces cytokines sur leur(s) récepteur(s).

Les composés anti-inflammatoires préférés selon l'invention sont les composés antagonistes de l'IL-1, de l'IL-8, du TNFα et du TNFβ.

Sont compris parmi les antagonistes des cytokines inflammatoires, selon l'invention le tripeptide Lys-Pro-Val (KPV), et tous les dérivés de l'αMSH, tous les inhibiteurs de libération des cytokines (classe thérapeutique des CSAIDs pour : cytokine suppressive anti-inflammatory drugs), la famille des pyrimidine N-Oxyde substitués (EP99401719.2 (priorité FR9809509) et EP 99402771.2 (priorité FR9814211) inducteurs de lipoxine A4, les extraits d'algues capable de moduler la production des cytokines par les kératinocytes comme le Phycosaccharide® commercialisé par la société CODIF, la Phlorogine®, commercialisée par la société SECMA, les extraits *d'Aloe vera* ou de *Gingko biloba* ou encore de *Rosa gallica* ; l'antagoniste naturel à l'IL-1 (II-1RA).

Selon l'invention, le composé ou agent anti-inflammatoire peut également choisi parmi les anti-inflammatoires stéroïdiens (Hydrocortisone, betaméthasone, dexaméthasone etc..) ou non stéroïdiens comme l'aspirine ou le paracetamol.

Selon l'invention, le dérivé anti-inflammatoire pourra également être choisi parmi des antagonistes de neuropeptides pro-inflammatoires (antagoniste de la substance P, antagoniste CGRP (pour calcitonin gene related peptide), antagoniste bradykinine) voire même des antagonistes d'AAEs (acides aminés excitateurs).

Les composés ou agents anti-inflammatoires sont de préférence présents dans les compositions conformes à l'invention à une concentration pouvant varier entre 0,00001 et 10 % en poids environ par rapport au poids total de la composition. Encore plus préférentiellement, la concentration en composé anti-inflammatoire peut varier entre 0.0005% et 2 % en poids par rapport au poids total de la composition

Les composés de formule (I) peuvent notamment être employés dans une composition qui comprend un milieu physiologiquement acceptable, notamment dans une composition cosmétique ou pharmaceutique qui comprend donc par ailleurs un milieu cosmétiquement ou pharmaceutiquement acceptable.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux. Il peut ainsi comprendre une phase aqueuse et/ou une phase grasse voire être incorporé dans des émulsions plus complexes comme les émulsions triples (e .g . eau dans huile dans eau) .

L'association d'au moins un composé N-acylamino-amide et d'au moins un composé anti-inflammatoire peut notamment être employée, seule ou en mélange, dans une composition qui comprend un milieu physiologiquement acceptable, notamment dans une composition cosmétique ou pharmaceutique qui comprend donc par ailleurs un milieu cosmétiquement ou pharmaceutiquement acceptable.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux. Il peut ainsi comprendre une phase aqueuse et/ou une phase grasse.

Pour une application sur la peau, la composition peut avoir la forme notamment de solution aqueuse ou huileuse; de dispersion du type lotion ou sérum; d'émulsions de consistance liquide ou semi-liquide du type lait obtenues par dispersion d'une phase grasse dans une phase aqueuse (H/E) ou inversement (E/H); de suspensions ou émulsions de consistance molle du type crème ou gel aqueux ou anhydres; de microcapsules ou microparticules; de dispersions vésiculaires de type ionique et/ou non ionique.

Pour une application sur les cheveux, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.
Ladite phase aqueuse peut comprendre en outre des alcools tels que des monoalcools en C₁-C₆ et/ou des polyols tels que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30% en poids par rapport au poids total de la composition. La composition selon l'invention peut également comprendre au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut également comprendre une phase grasse, notamment constituée de corps gras liquides à 25°C, tels que des huiles d'origine animale, végétale, minérale ou synthétique, volatiles ou non; de corps gras solides à 25°C tels que des cires d'origine animale, végétale, minérale ou synthétique; de corps gras pâteux; de gommes; de leurs mélanges.

Les huiles volatiles sont généralement des huiles ayant, à 25°C, une tension de vapeur saturante au moins égale à 0,5 millibar (soit 50 Pa).

Parmi les constituants de la phase grasse, on peut citer :
- les silicones volatiles cycliques ayant de 3 à 8 atomes de silicium, de préférence de 4 à 6.
- les cyclocopolymères du type diméthylsiloxane/méthylalkylsiloxane,
- les silicones volatiles linéaires ayant de 2 à 9 atomes de silicium.
- les huiles volatiles hydrocarbonées, telles que les isoparaffines et notamment l'isododécane et des huiles fluorées.
- les polyalkyl(C₁-C₂₀) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule R1COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.
- les gommes de silicones;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques ou pharmaceutiques hydrophiles ou lipophiles, les conservateurs, les antioxydants, les solvants, les parfums, les charges, les pigments, les nacres, les filtres UV, les absorbeurs d'odeur et les colorants. Ces adjuvants, selon leur nature, peuvent être introduits dans la phase grasse, dans la phase aqueuse et/ou dans des sphérules lipidiques.

La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

Les compositions cosmétiques ou pharmaceutiques selon l'invention peuvent notamment se présenter sous la forme d'une composition destinée au soin et/ou au traitement des zones ulcérées ou ayant subi un stress ou microstress cutané, notamment généré par une exposition aux UV et/ou la mise en contact avec un produit irritant.

Ainsi, les compositions selon l'invention peuvent notamment se présenter sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux; un shampooing antiparasitaire;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres;
- d'un produit d'hygiène buccale tel qu'une pâte dentifrice ou une lotion de rinçage buccal.
   Les compositions selon l'invention trouvent une application préférée comme composition de soin de la peau du visage, de type anti-rides ou anti-age, et comme composition de protection solaire ou après-soleil.
   La présente invention a également pour objet un procédé de traitement cosmétique de la peau du corps ou du visage, y compris le cuir chevelu, dans lequel on applique sur la peau une composition cosmétique comprenant une association entre un composé de la famille des N-acylamino-amides et au moins un agent anti-inflammatoire, à laisser celle-ci en contact puis éventuellement à rincer.
   Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs; application d'une lotion pour cuir chevelu sur cheveux mouillés; application de dentifrice sur les gencives.
   L'invention est illustrée plus en détail dans les exemples suivants.

### Example 1

### Préparation du {2-[acétyl-(3-trifluorométhyl-phényl)-amino]-3-méthylbutyrylamino} acétate d'éthyle de formule :

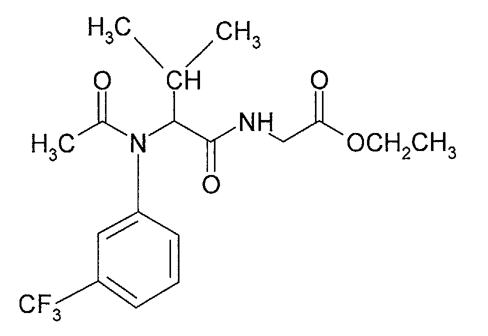

On mélange 0,63 ml d'isobutyraldéhyde et 1 ml de trifluorométhylamine (1,15 eq) dans 15 ml de méthanol, sous agitation. On laisse réagir 15 minutes à 20°C, puis on ajoute 0,46 ml d'acide acétique (1,15 eq) et on laisse réagir 10 minutes à 20°C. On ajoute alors 0,8 ml d'isocyanoacétate d'éthyle à 95% (1 eq) et on laisse réagir 48 heures à 20°C.

On concentre le milieu réactionnel au rotovapor et on purifie le résidu sur colonne de silice (éluant : heptane : 3 / acétate d'éthyle : 7; Rf = 0,5).

On obtient 2,45 g de composé sous forme de solide cireux, d'où un rendement de 91%.
RMN ¹H (200 MHz; CDCl3) δ ppm : 0,9 (6H;q), 1,3 (3H;t), 1,8 (3H;s), 2,3 (1H;m), 4,0 (2H,q), 4,2 (2H;q), 4,4 (2H;d), 7,3 (1H;t), 7,5 (4H;m)

### Example 2

### Préparation de l'acide {2-[acétyl-(3-trifluorométhyl-phényl)-amino]-3-méthyl-butyrylamino} acétique de formule :

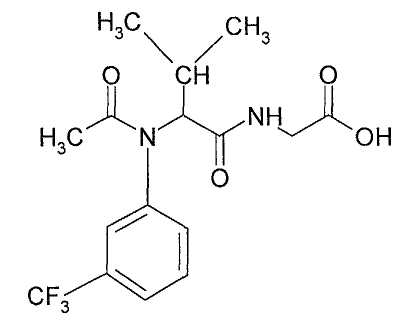

On solubilise 2 g de composé préparé à l'exemple 1 dans 30 ml d'acétone. On ajoute 30 ml de soude 2N et on laisse réagir pendant 6 heures à 20°C. On concentre le milieu réactionnel au rotovapor. On acidifie la phase aqueuse résiduelle à pH 2 par ajout d'HCI concentré puis on extrait par CH2Cl2.

La phase organique est concentrée à sec après séchage sur sulfate de sodium.

On obtient un résidu qui est solubilisé par un mélange eau basique à 10% d'éthanol, puis de nouveau acidifié par HCI concentré à pH 2. On extrait une nouvelle fois par Ch2Cl2 et on sèche la phase organique sur sulfate de sodium, on la filtre et on la concentre à sec sous vide au rotovapor.

On obtient 1,3 g de composé sous forme d'un solide légèrement marron clair, d'où un rendement de 70%.
RMN ¹H (200 MHz; DMSO) δ ppm : 0,9 (6H;q), 3,7 (2H;m), 1,8 (4H;m), 4,8 (2H;d), 7,6 (4H,m),8,4 (1H;t), 12,5 (1H;s)

### Example 3

On a déterminé in vitro l'activité anti-élastasique de composés selon l'invention, vis-à-vis de l'élastase leucocytaire humaine (ELH).

Le test est effectué de la manière suivante :

On laisse incuber, à 37°C, pendant 60 minutes, un substrat Me-OSAAPV-p-NA (Méthyl-O-succinate alanine alanine proline valine-p-nitroanilide) sur lequel est appliqué de l'ELH (40 milli-unités par ml) et 0,1% du composé à tester.

On détermine ensuite par spectrophotométrie le % d'inhibition de l'activité élastase témoin.

Les composés testés sont les suivants :
Composé A : acide {2-[acétyl-(3-trifluorométhyl-phényl)-amino]-3-méthylbutyryl-amino} acétique
Composé B : (2-{benzyl-[(diethoxy-phosphoryl)-acétyl]-amino}-3-méthylbutyryl-amino) acétate d'éthyle
Composé C : acide [2-(acétyl-benzyl-amino)-3-méthyl-butyrylamino] acétique
Composé D : [2-(acétyl-benzyl-amino)-3-méthyl-butyrylamino] acétate d'éthyle

On obtient les résultats suivants :

| Composé (concentration : 0,1%) | % d'inhibition de l'activité élastase témoin |
|---|---|
| Composé A | 67% |
| Composé B | 17% |
| Composé C | 20% |
| Composé D | 13% |

On détermine de la même manière le % d'inhibition de l'activité élastase témoin pour le composé A, à différentes concentrations.

On obtient les résultas suivants :

| Concentration du composé A | % d'inhibition de l'activité élastase témoin |
|---|---|
| 0,01% | 53% |
| 0,05% | 50% |
| 0,1% | 68% |
| 0,2% | 68% |

Le composé A provoque donc une forte inhibition de l'activité élastase, même en une quantité faible.

### Example 4

On a évalué l'activité ex vivo du composé de l'exemple 2 sur des peaux humaines en survie traitées par de l'élastase leucocytaire humaine (ELH).

Le test est effectué de la manière suivante :

Des coupes fraîches de peaux humaines, provenant de 2 donneurs différents, sont traitées pendant 2 heures, à 20°C, par 20 µl de solution tampon (pH 7,4) comprenant éventuellement 10 µg/ml d'ELH et éventuellement 0,1% du composé à tester, éventuellement préalablement mis en solution dans l'éthanol.

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques | |
|---|---|---|
| | Peau 1 | Peau 2 |
| Témoin (peau non traitée) | 12,7% | 15,25% |
| Peau traitée avec ELH | 4,85% | 6,85% |
| Peau traitée avec ELH + composé de l'exemple 2 | 13,95% | 11,85% |

On constate donc que le composé selon l'invention génère une protection significative des peaux vis-à-vis de la destruction des fibres élastiques induite par l'élastase.

### Example 5

On a évalué l'activité ex vivo du composé de l'exemple 2 sur des peaux humaines en survie traitées par de l'élastase leucocytaire humaine (ELH).

Le test est effectué de la manière suivante :

Des fragments de peau humaine normale provenant de trois donneurs différents sont déposées dans des inserts positionnés dans des puits de culture. On ajoute du milieu de culture supplémenté en antibiotiques dans le fond des puits. Un passage s'effectue par diffusion lente entre les deux compartiments par l'intermédiaire d'une membrane poreuse (taille des pores : 12 µm).

Le milieu de culture est renouvelé tous les trois jours.

On ajoute sur les fragments de peau éventuellement 0,5 µg d'ELH par ml de milieu de culture.

On ajoute également, tous les deux jours, 5 µl du composé à tester, préalablement mis en solution à 0,2% en poids dans l'éthanol.

Les peaux sont maintenues en survie pendant 10 jours à 37°C.

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques |
|---|---|
| Témoin (peau non traitée) | 7,4% |
| Peau traitée avec ELH | 5,1% |
| Peau traitée avec ELH + composé de l'exemple 2 | 7,1% |

On constate donc que le composé selon l'invention génère une protection significative des peaux vis-à-vis de la destruction des fibres élastiques induite par l'élastase.

### Example 6

On a évalué l'activité du composé de l'exemple 2 sur des peaux humaines en survie irradiées par des UVA (8 J/cm²).

Le test est effectué de la manière suivante :

Des fragments de peau humaine normale provenant de quatre donneurs différents sont déposées dans des inserts positionnés dans des puits de culture. On ajoute du milieu de culture supplémenté en antibiotiques dans le fond des puits. Un passage s'effectue par diffusion lente entre les deux compartiments par l'intermédiaire d'une membrane poreuse (taille des pores : 12 µm).

Le milieu de culture est renouvelé tous les trois jours.

On ajoute sur les fragments de peau, tous les deux jours, 5 µl du composé à tester, préalablement mis en solution à 0,2% dans l'éthanol.

Les peaux sont maintenues en survie pendant 7 jours à 37°C.

Les peaux sont irradiées une seule fois à 8 J/cm² (lampe Vilbert-Lourmat RMX-3W).

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | Analyse morphométrique des fibres élastiques (derme superficiel) | Analyse morphométrique du collagène (derme superficiel) |
|---|---|---|
| Peau non traitée | 6,75% | 87% |
| Peau traitée par des UVA (8 J/cm²) | 3,9% | 81% |
| Peau traitée par des UVA (8 J/cm²) + composé | 6,8% | 92% |

On constate que le composé selon l'invention a bien une activité vis-à-vis de la dégradation des fibres élastiques dans le derme superficiel de peaux irradiées par des UVA.

Ce composé présente également un effet adéquat sur la protection du collagène.

### Example 7 : Composition pour application topique.

- 4-N-Alkhyl pyrimidine N-Oxyde 0,1 %
- composé de l'Exemple 1 1 %
- isostéarate de propylène glycol 13 %
- polyéthylène glycol (8 OE) 5 %
- propylène glycol 3 %
- pentylène glycol 3 %
- stéarate de glycéryle et stéarate de polyéthylène glycol (100 OE) 5 %
- mono-stéarate de sorbitane oxyéthyléné (20 OE) 0,5 %
- alcool cétylique oxyéthyléné (20 OE) oxypropyléné (5 OP) 1 %
- gélifiants 0,5 %
- benzoates d'alkyle en C₁₂₋₁₅ 4 %
- éthanol 3 %
- hydroxyde de sodium 0,12 %
- conservateurs qs
- eau qsp 100 %

### Example 8 : composition pour application topique

On prépare l'émulsion suivante de façon classique (% en poids) :
- Hydrocortisone 0,1 %
- composé de l'Exemple 1 1 %
- isostéarate de propylène glycol 13 %
- polyéthylène glycol (8 OE) 5 %
- propylène glycol 3 %
- pentylène glycol 3 %
- stéarate de glycéryle et stéarate de polyéthylène glycol (100 OE) 5 %
- mono-stéarate de sorbitane oxyéthyléné (20 OE) 0,5 %
- alcool cétylique oxyéthyléné (20 OE) oxypropyléné (5 OP) 1 %
- gélifiants 0,5 %
- benzoates d'alkyle en C₁₂₋₁₅ 4 %
- éthanol 3 %
- hydroxyde de sodium 0,12 %
- conservateurs qs
- eau qsp 100 %

### Example 9 : crème de soin du visage

On prépare l'émulsion huile-dans-eau suivante de façon classique (% en poids) :
- Composé de l'exemple 2 1 %
- Stearate de glycérol 2 %
- Polysorbate 60 (Tween 60® vendu par la société ICI) 1 %
- Acide stéarique 1,4 %
- Triéthanolamine 0,7 %
- Carbomer 0,4 %
- Fraction liquide du beurre de karité 12 %
- Perhydrosqualène 12 %
- Extrait de *Rosa gallica* 1 %
- Antioxydant qs
- Parfum qs
- Conservateur qs
- Eau qsp 100 %

### Example 10 : lait pour le visage

On prépare le lait suivant de façon classique (% en poids) :
- Huile de vaseline 7 %
- Composé de l'exemple 2 1 %
- Monostéarate de glycéryle, stéarate de polyéthylène glycol (100 OE) 3 %
- Polymère carboxyvinylique 0,4 %
- Alcool stéarylique 0,7 %
- Protéines de soja 3 %
- NaOH 0,4 %
Tripeptide LPV 0.001 %
- Conservateur qs
- Eau qsp 100 %

### Example 11 : lotion capillaire antichute.

On prépare la lotion suivante de façon classique (% en poids) :
- composé de l'Exemple 1 1 %
- propylène glycol 23 %
- éthanol 55 %
minoxidil 2 %
Hydrocortisone 0.1%
- eau qsp 100 %

On peut appliquer cette lotion sur le scalp des individus alopéciques, pour prévenir les effets des UV, avant et/ou après exposition au soleil.

### Example 12 : lotion antichute

On prépare la lotion suivante de façon classique (% en poids) :
- composé de l'Exemple 2 1 %
- propylène glycol 23 %
- éthanol 55 %
- Aminexil 1,5 %
Tripeptide LPV 0.0005%
- eau qsp 100 %

On peut appliquer cette lotion antichute sur le scalp des individus alopéciques.

## Revendications

1. Composition cosmétique ou dermatologique **caractérisée en ce qu'**elle comprend une association entre un composé inhibiteur de l'élastase de la famille des N-Acylaminoamides et au moins un au moins un composé anti-inflammatoire.

2. Composition selon la revendication 1, **caractérisée en ce que** le composé , le composé inhibiteur de la famille des N-Acylaminoamides est un composé de formule (I) : 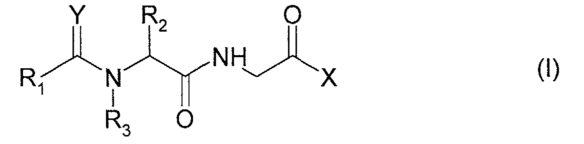 dans laquelle :
- le radical Y représente O ou S,
- le radical R1 représente :
- (i) un atome d'hydrogène;
- (ii) un radical hydrocarboné, linéaire, ramifié ou cyclique, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR; -P(O)-(OR)₂; -SO₂-OR;
avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- (iii) un radical choisi parmi les radicaux -OR; -NH₂; -NHR; -NRR'; -NH-COR; -COOR; -COR ;
avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R2 représente un radical hydrocarboné, linéaire, ramifié ou cyclique, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR;
avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R3 représente un radical choisi parmi ceux de formule (II) ou (III):
(II) -A-C₆H_{(5-y)}-B_{y}
(III) -C₆H_{(5-y')}-B_{y'}
dans lesquelles :
- y est un entier compris entre 0 et 5 inclus, et y' est un entier compris entre 1 et 5 inclus;
- A est un radical divalent hydrocarboné, linéaire ou ramifié, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- B est un radical hydrocarboné, linéaire ou ramifié, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical X représente un radical choisi parmi -OH, -OR₄, -NH₂, -NHR₄, -NR₄R₅ , -SR₄, -COOR₄; -COR₄;
avec R₄ et R₅ représentant, indépendamment l'un de l'autre, un radical hydrocarboné, linéaire, cyclique ou ramifié, saturé ou insaturé, ayant 1 à 6 atomes de carbone, éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné; lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
lesdits radicaux R₄ et R₅ pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
ses sels d'acide minéral ou organique, ses isomères optiques, sous forme isolée ou en mélange racémique.

3. Composition selon la revendication 2 dans laquelle le composé de formule (I) est tel que :
- le radical Y représente l'oxygène, et/ou
- le radical R1 représente l'hydrogène ou un radical hydrocarboné, linéaire ou ramifié, saturé ou insaturé, ayant 1 à 12, et notamment 1, 2, 3, 4, 5 ou 6, atomes de carbone, éventuellement substitué, et/ou
- les substituants de R1 sont choisis parmi -OH, -OR et/ou -P(O)-(OR)₂ avec R représentant un radical hydrocarboné, linéaire, ramifié ou cyclique, saturé ou insaturé, ayant 1 à 6 atomes de carbone, éventuellement halogéné, voire perhalogéné; et/ou
- le radical R2 représente un radical hydrocarboné, linéaire, ramifié ou cyclique, saturé ou insaturé, ayant 1 à 12, notamment 1, 2, 3, 4, 5 ou 6, atomes de carbone, éventuellement substitué; et/ou
- les substituants de R2 sont choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné; et/ou
- le radical R3 représente un radical de formule -C₆H_{(5-y')}-B_{y'} pour lequel y' = 1, 2 ou 3; ou un radical de formule -A-C₆H_{(5-y)}-B_{y} pour lequel y = 0, 1 ou 2; et/ou
- le radical A de R3 est un radical divalent hydrocarboné, linéaire ou ramifié, saturé ou insaturé, ayant 1 à 12 atomes de carbone, éventuellement substitué; et/ou
- le radical B de R3 est un radical hydrocarboné, linéaire ou ramifié, saturé ou insaturé, ayant 1 à 12 atomes de carbone, éventuellement substitué; et/ou
- les substituants de A et/ou de B sont choisis parmi -Hal (halogène, voire perhalogène); -CN; -COOR; -NO₂; -SO₂-OR; avec R représentant un radical hydrocarboné, linéaire, ramifié ou cyclique, saturé ou insaturé, ayant 1 à 6 atomes de carbone, éventuellement halogéné, voire perhalogéné; et/ou
- le radical X représente un radical choisi parmi -OH ou -OR₄ avec R₄ représentant un radical hydrocarboné, linéaire, cyclique ou ramifié, saturé ou insaturé, ayant 1 à 6 atomes de carbone, éventuellement substitué; et/ou
- les substituants de R4 de X sont choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné.

4. Composition selon l'une des revendications 2 ou 3, dans laquelle le composé de formule (I) est tel que :
- le radical R1 représente un radical méthyle, éthyle, propyle ou isopropyle, éventuellement substitué par un groupement -OH ou -P(O)-(OR)₂ avec R représentant méthyle, éthyle, propyle ou isopropyle; et/ou
- le radical R2 représente un radical méthyle, éthyle, propyle, isopropyle, n-butyle, ter-butyle ou isobutyle; et/ou
- le radical R3 représente un groupement choisi parmi l'une des formules suivantes : 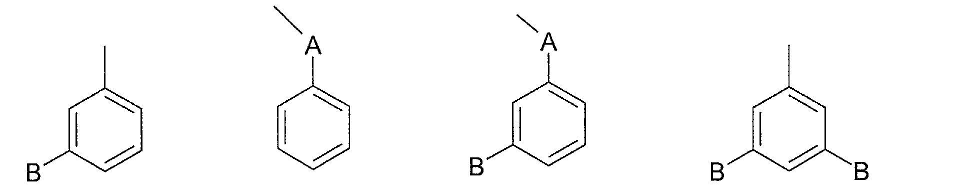 dans lesquelles le radical divalent A est un méthylène, un éthylène, un propylène et/ou le radical B est un radical méthyle, éthyle, propyle ou isopropyle, substitué par un ou plusieurs halogènes, notamment chlore, brome, iode ou fluor, et préférentiellement totalement halogéné (perhalogéné), tel que perfluoré, notamment le radical perfluorométhyle (-CF3).
- le radical X représente un radical choisi parmi -OH, -OCH₃, -OC₂H₅, -O-C₃H₇ ou -OC₄H₉.

5. Composition selon l'une des revendications 2 à 4, dans laquelle le composé de formule (I) est choisi parmi les composés suivants :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle,
- le (2-{benzyl-[(diethoxy-phosphoryl)-acétyl]-amino}-3-méthyl-butyrylamino) acétate d'éthyle.

6. Composition selon l'une des revendications précédentes, dans laquelle le composé inhibiteur de l'élastase de la famille des N-Acylaminoamides est présent en une quantité comprise entre 0,00001 et 20% en poids par rapport au poids total de la composition, préférentiellement entre 0,001 et 10% en poids.

7. Composition selon l'une des revendications précédentes, **caractérisée ne ce que** le composé anti-inflammatoire est choisi parmi les composés antagonistes des cytokines pro-inflammatoires comme l'IL-1, le TNFα et le TNFβ ou les chemokines comme l'IL-8 ou le MCAF (facteur activateur et chimiotactique des macrophages).

8. Composition selon l'une des revendications 1 à 6, **caractérisé en ce que** le composé anti-inflammatoire est choisi parmi les inhibiteurs de libération des cytokines et les inducteurs de lipoxine A4.

9. Composition selon l'une des revendications 1 à 6, **caractérisé en ce que** le composé anti-inflammatoire est choisi parmi le tripeptide Lys-Pro-Val et les dérivés de l'αMSH.

10. Composition selon l'une des revendications 1 à 6, **caractérisé en ce que** le composé anti-inflammatoire est choisi parmi les composés extraits d'algues capables de moduler la production des cytokines par les kératinocytes, comme le Phycosaccharide, la Phlorogine, ou les extraits *d'Aloe vera* ou de *Gingko biloba* ou de *Rosa gallica*.

11. Composition selon l'une des revendications 1 à 6, **caractérisé en ce que** le composé anti-inflammatoire est un composé de la famille des pyrimidine N-Oxyde substitués.

12. Composition selon l'une des revendications 1 à 6, **caractérisé en ce que** le composé anti-inflammatoire est choisi parmi les anti-inflammatoires stéroïdiens, comme l'hydrocortisone, la betamethasone et la dexamethasone.

13. Composition selon l'une des revendications 1 à 6, **caractérisé en ce que** le composé anti-inflammatoire est choisi parmi les anti-inflammatoires non stéroïdiens, comme l'aspirine ou le paracetamol.

14. Composition selon l'une des revendications 1 à 6, **caractérisé en ce que** le composé anti-inflammatoire est choisi parmi des antagonistes de neuropeptides pro-inflammatoires (comme la substance P, le CGRP et la bradykinine).

15. Composition selon l'une des revendications 1 à 6, **caractérisé en ce que** le composé anti-inflammatoire est choisi parmi des antagonistes d'acides aminés excitateurs.

16. Composition selon l'une des revendications 1 à 15 **caractériséd en ce que** le composé anti-inflammatoire est présent en une quantité comprise entre 0,00001 % et 10 % en poids par rapport au poids total de la composition, préférentiellement entre 0,0005 et 2 % en poids.

17. Composition selon l'une des revendications précédentes, se présentant sous la forme d'une composition cosmétique ou dermatologique destinée au soin et/ou au traitement des zones ulcérées ou ayant subi un stress ou microstress cutané, notamment généré par une exposition aux UV et/ou la mise en contact avec un produit irritant.

18. Composition selon l'une des revendications 1 à 15, se présentant sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres;
- d'un produit d'hygiène buccale tel qu'une pâte dentifrice ou une lotion de rinçage buccal.

19. Composition selon l'une des revendications 1 à 15, se présentant sous la forme d'une composition de soin de la peau du visage, de type anti-rides ou anti-age, ou d'une composition de protection solaire ou après-soleil.

20. Procédé de traitement cosmétique de la peau du corps ou du visage, y compris le cuir chevelu, dans lequel on applique sur la peau une composition cosmétique telle que définie dans l'une des revendications 1 à 18.
